Europäisches Patentamt

European Patent Office

Office européen des brevets

(11) Veröffentlichungsnummer: **0 510 626 A1**

## EUROPÄISCHE PATENTANMELDUNG

(21) Anmeldenummer: **92106922.5**

(51) Int. Cl.⁵: **A61M 5/32**

(22) Anmeldetag: **23.04.92**

(30) Priorität: **25.04.91 DE 4113456**

(43) Veröffentlichungstag der Anmeldung:
**28.10.92 Patentblatt 92/44**

(84) Benannte Vertragsstaaten:
**AT BE CH DE DK ES FR GB GR IT LI LU MC NL PT SE**

(71) Anmelder: **HEROTEC KUNSTSTOFFVERARBEITUNG GmbH Am Bosenberg 7**
**W-4730 Ahlen-Vorhelm(DE)**

(72) Erfinder: **Battert, Günter**
**Am Elsawäldchen 1a**
**W-4722 Ennigerloh(DE)**

(74) Vertreter: **Patentanwälte Meinke und Dabringhaus Dipl.-Ing. J. Meinke Dipl.-Ing. W. Dabringhaus Westenhellweg 67 W-4600 Dortmund 1(DE)**

(54) **Vorrichtung zur Halterung der Schutzkappe einer Injektionsnadel.**

(57) Mit einer Vorrichtung zur Halterung der Schutzkappe für eine Nadel einer Injektionsspritze od. dgl. mit einem Trageteil (1), mit einer Aufnahme (2) für die Schutzkappe (3) und eine im Bereich der Aufnahme (2) wirksamen Klemmeinrichtung zur Halterung der Schutzkappe (3) in der Aufnahme (2), soll die gattungsbildende Einrichtung so verbessert werden, daß trotz leichter Bedienbarkeit eine sichere Festlegung auch unterschiedlich großer und glatter Schutzkappen in und an der Haltevorrichtung erreichbar ist.

Dies wird dadurch erreicht, daß die Klemmeinrichtung von einem in den durch die Aufnahme (2) gebildeten Raum verschiebbaren Klemmelement gebildet ist.

FIG. 1

EP 0 510 626 A1

Die Erfindung betrifft eine Vorrichtung zur Halterung der Schutzkappe für eine Nadel einer Injektionsspritze od. dgl. mit einem Trageteil, mit einer Aufnahme für die Schutzkappe und einer im Bereich der Aufnahme wirksamen Klemmeinrichtung zur Halterung der Schutzkappe in der Aufnahme.

Injektionsspritzen bestehen z.B. aus dem Zylinder mit dem handbetätigbaren Kolben, einer auf das vordere Ende des Zylinders aufsetzbaren Nadel und einer die Nadel umgebenden und schützenden Schutzkappe, die natürlich vor Durchführen der Injektion abgenommen wird. Nach Durchführen der Injektion wird das kontaminierte Teil einer Injektionsspritze, nämlich die Nadel, wieder zurück in die Schutzkappe gegeben und dann mit der schutzkappe zusammen entsorgt. Ein Problem bei dieser Rückgabe der Nadel in die Schutzkappe besteht darin, daß die Schutzkappe üblicherweise mit zwei Fingern festgehalten wird und dann die Nadel in die Aufnahmeöffnung der Schutzkappe eingeführt wird, wobei es immer wieder vorkommt, daß sich die diese Arbeit durchführende Person mit der Nadel sticht. Hier besteht erhebliche Infektionsgefahr und es ist aus diesem Grunde bereits Vorschrift, daß solche Vorgänge in einem entsprechenden Buch festgehalten werden, um so später entsprechende Verletzungen rekonstruieren zu können. Dieses Problem ist um so größer, je länger die Injektionsnadel ist.

Aus der gattungsbildenden US-PS 4 950 015 ist eine Haltevorrichtung bekanntgeworden, die mit einem federbeaufschlagten Stößel arbeitet, der an seinem Ende eine Schlaufe aufweist, die die Schutzkappe aufnehmen kann. Bei entsprechender Betätigung bewirkt die Feder ein Festklemmen der Schutzkappe zwischen der Schlaufe und einem Halteteil. Die die Festlegung bewirkende Feder muß dabei hinsichtlich ihrer Federkraft einerseits so leicht eingestellt sein, daß eine leichte Betätigung möglich ist, andererseits muß die Feder so stark wirken, daß ein sicheres Festlegen der Schutzkappe in der Schlaufe erreicht wird.

Aufgrund der Durchmesserunterschiede der Schutzkappen und aufgrund des gleitfähigen Werkstoffes zur Herstellung dieser Schutzkappe, ist eine solche sichere Festlegung mit dieser bekannten Einrichtung nicht möglich. Eine klammerartige Vorrichtung zur Halterung der Schutzkappe ist aus der US-PS 4 938 514 bekanntgeworden. Auch hier ist keine zufriedenstellende Halterung der Schutzkappe zu erreichen.

Man hat auch gemäß dem Vorschlag der US-PS 4 596 562 versucht, die Schutzkappe in einer einfachen Steckvorrichtung festzulegen, die unterschiedlich große Bohrungen zur Anpassung an die unterschiedlich großen Schutzkappen aufweist.

Auch diese Vorrichtung arbeitet keineswegs zufriedenstellend und sicher.

Der Erfindung liegt die Aufgabe zugrunde, die gattungsbildende Einrichtung so zu verbessern, daß trotz leichter Bedienbarkeit eine sichere Festlegung auch unterschiedlich großer und glatter Schutzkappen in und an der Haltevorrichtung erreichbar ist.

Diese Aufgabe wird mit einer Vorrichtung der eingangs bezeichneten Art erfindungsgemäß dadurch gelöst, daß die Klemmeinrichtung von einem in den durch die Aufnahme gebildeten Raum verschiebbaren Klemmelement gebildet ist.

Mit der Erfindung ist es möglich, bei leichter Bedienbarkeit eine sichere Festlegung auch unterschiedlich großer und glatter Schutzkappen in und an der Haltevorrichtung zu erreichen, da mittels des Klemmelementes die Aufnahme an die Größe der jeweiligen Schutzkappe auf einfache Weise anpaßbar ist.

In vorteilhafter Ausgestaltung der Erfindung ist vorgesehen, daß das Klemmelement als von einer Schraubspindel getriebener Klemmbolzen ausgebildet ist, wobei bevorzugt die Schraubspindel über einen Rändelkopf betätigbar ist. Nach dieser Ausgestaltung wird somit ein über einen Rändelkopf bewegbarer Klemmbolzen vorgeschlagen, wobei der entsprechende untere Teil des Klemmbolzens als Schraubspindel ausgebildet ist.

Alternativ schlägt die Erfindung vorteilhaft vor, daß das Klemmelement von einer Feder in Klemmrichtung getrieben und über ein Handhabungselement im Trageteil aus der Klemmstellung in eine freigebende Stellung bewegbar ist. Mit dieser Ausgestaltung ist eine besonders einfache Anpassung und Festlegung der Schutzkappe in der Aufnahmevorrichtung möglich, vor dem Einführen der Schutzkappe muß lediglich das Klemmelement aus der Aufnahme herausbewegt werden, anschließend wird dieses federbelastet selbsttätig zurückbewegt und fixiert die Schutzkappe.

Besonders vorteilhaft sieht die Erfindung auch vor, daß im Trageteil ein Ampullenmesser und/oder eine Ampullenabbrechöffnung vorgesehen ist. Durch diese zusätzliche Ausgestaltung wird die Anwendungsmöglichkeit der Vorrichtung noch vergrößert, die Vorrichtung kann auch zum Öffnen bzw. Abbrechen von Ampullen eingesetzt werden, wodurch eine Verletzung des Bedienungspersonales auch beim öffnen der Ampullen zuverlässig vermieden werden kann, da das Bedienungspersonal nicht mit der Ampullenschnittfläche direkt in Kontakt gerät.

Die Erfindung sieht ferner vorteilhaft vor, daß am Trageteil ein Klemmbügel angeordnet ist. Die Vorrichtung kann dann wie ein Kugelschreiber vom Bedienungspersonal in einer Kleidungstasche mitgeführt werden und ist jederzeit verfügbar.

Die Erfindung ist nachstehend anhand der Zeichnung beispielsweise näher erläutert. Diese zeigt in

Fig. 1 eine Ausbildung der Halterungsvorrichtung in einer auseinandergezogenen Darstellungsweise,

Fig. 2 die Halterungsvorrichtung gemäß Fig. 1 im eingesetzten Zustand,

Fig. 3 in einer Schnittdarstellung eine Klemmvorrichtung mit Klemmbolzen und in

Fig. 4 in einer Draufsicht eine andere Ausführungsform einer erfindungsgemäßen Halterungsvorrichtung.

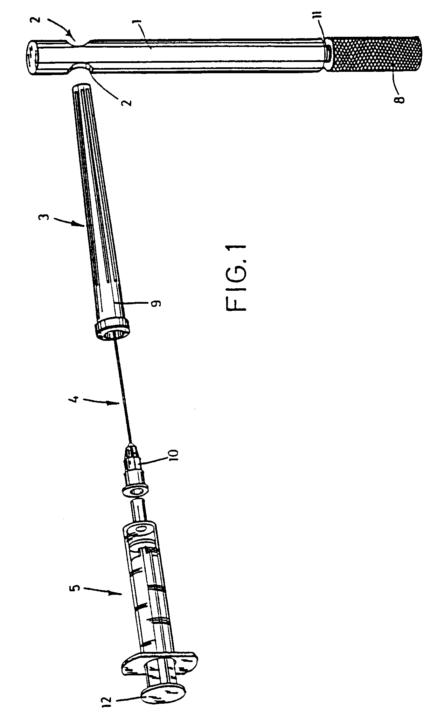

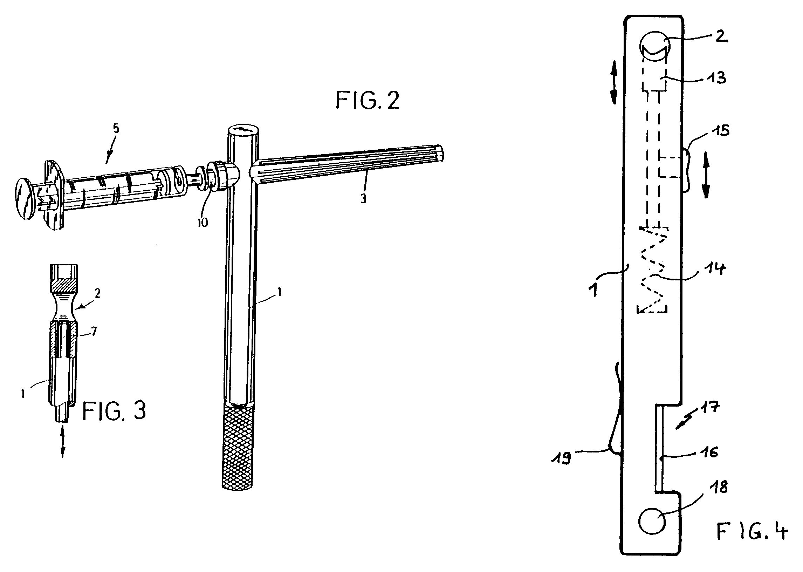

In den Zeichnungen ist ein Trageteil 1 gezeichnet, das gemäß Fig. 1 und 2 aus einem zylindrischen, etwa die Größe eines Füllfederhalters aufweisenden Bauteil besteht, das in seinem unteren Rand einen Rändelkopf 8 aufweist, der mit einer Schraubspindel 11 in Verbindung steht. Am gegenüberliegenden Ende des Trageteiles 1 ist eine Aufnahme 2 vorgesehen, die in ihrer Form und Größe den üblichen Abmessungen einer Schutzkappe 3 angepaßt ist. Die Schutzkappe 3 gehört zu einem Einweg-Nadelset, der durch diese Schutzkappe 3 und eine Nadel 4 gebildet wird. Die Schutzkappe 3 ist an ihrem einen Ende geschlossen ausgebildet und weist an dem anderen Ende eine Aufnahmebuchse 9 auf, die mit einem Kopfteil 10 der Nadel 4 derart zusammenwirken kann, daß hier ein Festklemmen der Nadel 4 in der Schutzkappe erfolgen kann.

Bei 5 ist eine Zylinderkolbenanordnung dargestellt, wodurch die eigentliche Injektionsspritze gebildet wird. Das untere, zylindrisch ausgebildete Ende dieser Zylinderkolbenanordnung 5 kann mit dem Kopfteil 10 der Nadel in Verbindung gebracht werden, während das gegenüberliegende Ende durch den Handbetätigungsteil 12 des Kolbens gebildet wird.

Fig. 2 zeigt, wie die Schutzkappe 3 in der Aufnahme 2 festgelegt ist und wie die Zylinderkolbenanordnung 5 auf das Kopfteil 10 der Nadel 4 aufgesetzt ist. Nunmehr kann die Zylinderkolbenanordnung 5 zusammen mit der Nadel 4 entnommen werden. Nach Durchführen der Injektion wird die Nadel 4 zurück in die fest in dem Trageteil 1 gehaltene Schutzkappe 3 eingeführt, wobei das Trageteil 1 dabei an seinem unteren Ende im Bereich des Rändelkopfes 8 gehalten wird, so daß ein Verletzen der Hand oder der Finger durch die in die Schutzkappe 3 einzusetzende Nadel 4 nicht erfolgen kann.

Das Festklemmen erfolgt durch einen Klemmbolzen 7, der am oberen Ende der Schraubspindel 11 angeordnet ist, die über den Rändelkopf 8 betätigt wird, so daß durch Betätigen des Rändelkopfes 8 der Klemmbolzen 7 die Außenseite der Schutzkappe 3 freigibt oder festklemmt.

In Fig. 4 ist eine andere Ausführungsform der erfindungsgemäßen Vorrichtung dargestellt. Das Trageteil 1 ist stabförmig ausgebildet und weist ebenfalls eine Aufnahme 2 für die Schutzkappe 3 auf, wobei das Klemmelement 13 in dieser Ausführungsform verschiebbar im Trageteil 1 angeordnet ist und von einer im Trageteil 1 angeordneten Feder 14 in Klemmrichtung, d.h. in Richtung der Aufnahme 2 getrieben wird. Das Klemmelement 13 ist mit einem Handhabungselement 15 verbunden, das an der Außenseite des Trageteiles 1 verschiebbar angeordnet ist. Mit diesem Handhabungselement 15 läßt sich das Klemmelement 7 aus der Klemmstellung in eine freigebende Stellung bewegen, um eine Schutzkappe 3 einzusetzen. Beim Loslassen des Handhabungselementes 15 wird dann das Klemmelement 13 durch die Feder 14 selbsttätig in die Aufnahme 2 gedrückt und legt die Schutzkappe 3 in dieser fest.

Zusätzlich sind am Trageteil 1 ein Ampullenmesser 16 in einer Aussparung 17 sowie eine Ampullenabbrechöffnung 18 vorgesehen. Die Vorrichtung eignet sich dann auch zum Öffnen und Abbrechen von Ampullen, wobei zunächst die Ampulle an einem Ende mit dem Ampullenmesser 16 angeritzt und anschließend durch Einführen in die Öffnung 18 und anschließendes Abwinkeln abgebrochen wird. Dabei ist zuverlässig vermieden, daß die Bedienungsperson mit der Schnittkante direkt in Verbindung kommt, so daß Verletzungen weitgehend ausgeschlossen werden.

Ferner ist am Trageteil 1 auch noch ein Klemmbügel 19 angeordnet, der wie bei einem Kugelschreiber od. dgl. ausgebildet ist und eine Aufbewahrung der Vorrichtung in einer Kitteltasche od. dgl. ermöglicht, so daß das Bedienungspersonal die Vorrichtung immer mit sich führen kann und diese jederzeit zur Verfügung hat.

Natürlich ist die Erfindung nicht auf die dargestellten Ausführungsbeispiele beschränkt. Weitere Ausgestaltungen der Erfindung sind möglich, ohne den Grundgedanken zu verlassen. So kann auch die in Fig. 1 und 2 dargestellte Vorrichtung mit einem entsprechenden Ampullenmesser oder einer Ampullenabbrechöffnung ausgestattet sein u. dgl. mehr.

**Patentansprüche**

1. Vorrichtung zur Halterung der Schutzkappe für eine Nadel einer Injektionsspritze od. dgl. mit einem Trageteil (1), mit einer Aufnahme (2) für die Schutzkappe (3) und einer im Bereich der Aufnahme (2) wirksamen Klemmeinrichtung zur Halterung der Schutzkappe (3) in der Aufnahme (2),
   dadurch gekennzeichnet,
   daß die Klemmeinrichtung von einem in den durch die Aufnahme (2) gebildeten Raum verschiebbaren Klemmelement (7,13) gebildet ist.

2. Vorrichtung nach Anspruch 1,
   dadurch gekennzeichnet,
   daß das Klemmelement als von einer Schraubspindel (11) getriebener Klemmbolzen (7) ausgebildet ist.

3. Vorrichtung nach Anspruch 2,
   dadurch gekennzeichnet,
   daß die Schraubspindel (11) über einen Rändelkopf (8) betätigbar ist.

4. Vorrichtung nach Anspruch 1,
   dadurch gekennzeichnet,
   daß das Klemmelement (13) von einer Feder
   (14) in Klemmrichtung getrieben und über ein
   Handhabungselement (15) im Trageteil (1) aus
   der Klemmstellung in eine freigebende Stellung bewegbar ist.

5. Vorrichtung nach Anspruch 1 oder einem der
   folgenden,
   dadurch gekennzeichnet,
   daß im Trageteil (1) ein Ampullenmesser (16)
   vorgesehen ist.

6. Vorrichtung nach Anspruch 1 oder einem der
   folgenden,
   dadurch gekennzeichnet,
   daß im Trageteil (1) eine Ampullenabbrechöffnung (18) vorgesehen ist.

7. Vorrichtung nach Anspruch 1 oder einem der
   folgenden,
   dadurch gekennzeichnet,
   daß am Trageteil (1) ein Klemmbügel (19) angeordnet ist.

FIG.1

EP 0 510 626 A1

FIG.2

FIG.3

FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | FR-A-2 586 566 (PUTSCHER) | 1 | A61M5/32 |
|  | * Seite 2, Zeile 7 - Zeile 24; Abbildungen 1,4 * |  |  |
| Y |  | 2-7 |  |
|  | --- |  |  |
| Y,P | US-A-5 013 299 (CLARK) | 2,3 |  |
|  | * Spalte 4, Zeile 37 - Zeile 51; Abbildungen 2,4 * |  |  |
|  | --- |  |  |
| Y | FR-A-2 639 235 (KINNEL) | 4 |  |
|  | * Seite 3, Zeile 84 - Zeile 89; Abbildungen 1,2 * |  |  |
|  | --- |  |  |
| Y | DE-U-9 002 469 (MINNINGER) | 5,6 |  |
|  | * Seite 6, Zeile 33 - Seite 7, Zeile 4; Abbildungen 5,6 * |  |  |
|  | --- |  |  |
| Y | US-A-4 950 015 (NEJIB ET AL.) | 7 |  |
|  | * Spalte 2, Zeile 21 * |  |  |
|  | * Spalte 2, Zeile 15; Abbildungen 3,5 * |  |  |
| A |  | 4 |  |
|  | --- |  | **RECHERCHIERTE SACHGEBIETE (Int. Cl.5 )** |
| A | US-A-4 846 803 (EMERSON) | 2 |  |
|  | * Spalte 3, Zeile 18 - Zeile 21; Abbildung 1 * |  | A61M |
|  | ----- |  |  |

Der vorliegende Recherchenbericht wurde für alle Patentansprüche erstellt

| Recherchenort | Abschlußdatum der Recherche | Prüfer |
|---|---|---|
| DEN HAAG | 04 AUGUST 1992 | SEDY R. |

EPO FORM 1503 03.82 (P0403)